# FASCICULE DE BREVET EUROPEEN

(11) **EP 3 027 312 B1**
(45) Date de publication et mention de la délivrance du brevet: **08.01.2020**
(21) Numéro de dépôt: 14745194.2
(22) Date de dépôt: 30.06.2014
(51) Int. Cl.: B01J 29/70, B01J 29/72, B01J 29/74, C01B 39/02, C01B 39/48, C07C 15/08, B01J 37/06, B01J 35/00, B01J 35/02, B01J 35/10, B01J 37/00, C07C 5/27

(54) **MODIFICATION DE ZEOLITHE DE TYPE EUO ET SON UTILISATION EN ISOMERISATION DES COMPOSES C8 AROMATIQUES**
MODIFIZIERUNG VON ZEOLITH VOM EUO-TYP ZEOLITH UND VERWENDUNG DAVON IN DER ISOMERISIERUNG VON AROMATISCHEN C8-VERBINDUNGEN
MODIFICATION OF EUO TYPE ZEOLITE AND USE THEREOF IN ISOMERIZATION OF AROMATIC C8 COMPOUNDS

(30) Priorité: 02.08.2013 FR 1357715
(43) Date de publication de la demande: 08.06.2016
(73) Titulaire: IFP Energies nouvelles, 92500 Rueil-Malmaison (FR)
(72) Inventeur: LAGAUCHE, Matthieu, F-69390 Vernaison (FR); BRANDHORST, Laure, F-69008 Lyon (FR); BOUCHY, Christophe, F-69007 Lyon (FR)
(74) Mandataire: IFP Energies nouvelles
(86) Numéro de dépôt international: PCT/FR2014/051671
(87) Numéro de publication internationale: WO 2015/015077

(56) Documents cités:
- EP-A1- 1 013 606
- EP-A1- 1 151 964
- FR-A1- 2 915 112
- FR-A1- 2 958 297
- US-A- 6 051 129

## Description

La présente invention se rapporte à un procédé de préparation d'un catalyseur comprenant une zéolithe de type structural EUO modifiée, au moins une matrice et au moins un métal du groupe VIII de la classification périodique des éléments. L'invention porte également sur l'utilisation dudit catalyseur préparé selon l'invention dans un procédé d'isomérisation d'une coupe aromatique comprenant au moins un composé aromatique à huit atomes de carbone par molécule pour la production de paraxylène. Cette coupe est communément appelée "coupe C8 aromatiques".

### État de la technique antérieure

Les catalyseurs utilisés pour la mise en œuvre d'un procédé d'isomérisation de composés aromatiques à huit atomes de carbone sont généralement des catalyseurs zéolithiques. Parmi les zéolithes utilisées en isomérisation d'une coupe C8 aromatiques, on trouve la ZSM-5, utilisée seule ou en mélange avec d'autres zéolithes, comme par exemple la mordénite. Ces catalyseurs sont notamment décrits dans les brevets US-A-4 467 129, US-A-4 482 773 et EP-B-13 617. Les catalyseurs de l'état de la technique, en particulier les catalyseurs à base de zéolithe mordénite présentent des performances catalytiques moyennes car ils conduisent à des réactions parasites non négligeables. Parmi ces réactions secondaires, on peut citer l'ouverture de cycles naphténiques suivie ou non de craquage (transformation vers les paraffines) ou encore les réactions de dismutation et de transalkylation des aromatiques à huit atomes de carbone (transformation vers des composés aromatiques non recherchés) ou bien encore l'hydrogénation des composés aromatiques (transformation vers les naphtènes). Des catalyseurs à base de zéolithe ZSM-5, seule ou en mélange avec d'autres zéolithes comme la mordénite, ont déjà été utilisés et n'atteignent pas des performances catalytiques optimales pour la conversion de l'éthylbenzène en xylènes.

Les demandes de brevet EP923 987A1 et WO2005/065.380 proposent respectivement un catalyseur à base d'une zéolithe de type structural EUO et de type structural MTW. Ces catalyseurs zéolithiques, bien que conduisant à une sélectivité en xylènes intéressante présentent un rendement en xylènes trop faible.

Le document EP 1 013 606 décrit la préparation de catalyseurs à base de zéolite de type boro-aluminosilicate, et de structures EUO et NES, pour leurs utilisations dans des procédés d'isomérisation de wax.

Le document EP 1 151 964 décrit un procédé de préparation d'une zéolithe de type structural EUO contenant un cation organique azoté du type dibenzyldimethyammonium et en présence de germe d'au moins un matériau zéolithique. La zéolithe est utilisée pour l'isomérisation de composés aromatiques à 8 atomes de carbone.

Il existe donc toujours un besoin de développer des catalyseurs d'isomérisation de coupe C8 aromatiques en xylènes présentant des performances améliorées en termes de sélectivité et d'activité.

Il est connu que l'activité du catalyseur peut être améliorée par la création de mésopores au sein et/ou entre les cristallites de la zéolithe utilisée (mésoporosité intra et/ou extra-cristalline). La désilication de la zéolithe en milieu alcalin constitue l'une des techniques utilisables pour générer de la mésoporosité. Cependant, bien que ce traitement soit connu de l'homme du métier, les conditions opératoires à mettre en œuvre sont propres aux caractéristiques spécifiques de chaque zéolithe (type structural, taille et morphologie des cristallites, rapport Si/Al etc...). Dans la littérature ouverte, la réalisation de désilication en milieu alcalin a été décrite pour des zéolithes de type structural MFI, MTW, MOR, BEA, AST, FER, MWW, IFR, STF, CHA, FAU et TON (D. Verboekend, J. Perez-Ramirez, Catalysis Science and Technology, 2011, 1, 897-890) cependant aucun document de la littérature ne décrit le traitement alcalin d'une zéolithe de type structural EUO.

La demanderesse dans ses recherches a mis au point un nouveau procédé de préparation d'un nouveau catalyseur comprenant une zéolithe de type structural EUO, plus particulièrement une zéolithe EU-1, modifiée par un traitement alcalin spécifique. Il a été constaté de façon surprenante, qu'un catalyseur, sous forme de billes ou d'extrudés, comprenant au moins une matrice, au moins un métal du groupe VIII de la classification périodique des éléments, et au moins une zéolithe EUO, plus particulièrement une zéolithe EU-1 ayant subi un traitement alcalin dans les conditions décrites par l'invention, conduit à des performances catalytiques améliorées pour l'isomérisation d'une charge aromatique lorsque ledit catalyseur est utilisé dans un procédé d'isomérisation d'une coupe aromatique comprenant au moins un composé aromatique à huit atomes de carbone par molécule. Un tel catalyseur est plus actif et plus sélectif qu'un catalyseur comprenant une zéolithe EUO n'ayant pas subi de traitement alcalin selon l'invention ou ayant subi un traitement alcalin dans des conditions différentes de celles décrites dans la présente invention. Il en résulte une augmentation du rendement en xylènes lorsque le procédé d'isomérisation est mis en oeuvre en présence du catalyseur selon l'invention.

### Description détaillée de l'invention

La présente invention porte sur procédé de préparation d'un catalyseur d'isomérisation d'une coupe aromatique contenant au moins un composé aromatique ayant 8 atomes de carbone par molécule, ledit catalyseur comprenant au moins une zéolithe EUO modifiée ayant subi un traitement alcalin, au moins une matrice et au moins un métal du groupe VIII de la classification périodique des éléments.

Le procédé selon l'invention est un procédé de préparation du catalyseur d'isomérisation d'une coupe aromatique contenant au moins un composé aromatique ayant 8 atomes de carbone par molécule à partir d'une zéolithe initiale de type structural EUO ayant un rapport atomique Si/Al global compris entre 5 et 100, ledit catalyseur comprenant au moins une matrice et au moins un métal du groupe VIII, ledit procédé comprenant au moins les étapes suivantes :
i) une étape de désilication de ladite zéolithe initiale de type structural EUO par suspension dans une solution alcaline ayant une normalité comprise entre 0,5 et 2 M, une agitation avec une vitesse d'agitation comprise entre 100 et 1000 tr/min, et un chauffage à une température comprise entre 40 et 100°C pendant une durée comprise entre 5 min et 24 h,
ii) une étape de lavage à l'eau distillée de la zéolithe issue de l'étape i),
iii) une étape de préparation du support par mise en forme de ladite zéolithe EUO lavée issue de l'étape ii) avec une matrice de sorte que le pourcentage poids de la zéolithe soit compris entre 3 et 80% par rapport au poids du support,
iv) au moins une étape d'échange ionique de la zéolithe lavée issue de l'étape ii) ou du support issu de l'étape iii) de manière à éliminer au moins en partie le cation alcalin présent en position cationique dans la zéolithe,
v) une étape de dépôt d'au moins un métal du groupe VIII de la classification périodique des éléments sur ladite zéolithe échangée issu de l'étape iv) ou ledit support échangé issu de l'étape iv) de sorte que le pourcentage poids dudit métal soit de 0,01 à 4% par rapport au poids du catalyseur,
procédé dans lequel la matrice pour la mise en forme du support est choisie parmi les argiles, la magnésie, les alumines, les silices, l'oxyde de titane, l'oxyde de bore, la zircone, les phosphates d'aluminium, les phosphates de titane, les phosphates de zirconium, les silices-alumines et le charbon ou un mélange d'au moins deux de ces compositions,
et dans lequel la solution alcaline est une solution aqueuse alcaline à base d'hydroxyde de lithium LiOH, d'hydroxyde de sodium NaOH, d'hydroxyde de potassium KOH ou d'hydroxyde de césium CsOH.

### i) Etape de désilication

La zéolithe initiale de type structural EUO initiale utilisée dans la présente invention peut être choisie parmi la zéolithe EU-1, la zéolithe TPZ-3 et la zéolithe ZSM-50, de préférence ladite zéolithe est la zéolithe EU-1.

Les zéolithes EU-1, TPZ-3 et ZSM-50 de type structural EUO sont bien connues de l'art antérieur (Atlas of Zeolite Framework Types, Ch. Baerlocher, W.M. Meier, D.H. Oison, 5ème édition, 2001). Il est connu qu'une zéolithe de type structural EUO, en particulier une zéolithe EU-1, présente un réseau microporeux monodimensionnel, dont le diamètre des pores est de 4,1 x 5,4 Å (1 Å = 1 Angström = 10⁻¹⁰ m). D'autre part, N.A. Briscoe et al. ont enseigné dans un article de la revue Zeolites (1988, 8, 74) que ces canaux monodimensionnels possèdent des poches latérales de profondeur 8,1 Å et de diamètre 6,8 x 5,8 Å.

La zéolithe initiale de type structural EUO utilisée dans la présente invention présente un rapport atomique Si/Al global compris entre 5 et 100, de préférence compris entre 10 et 50 et de manière très préférée compris entre 10 et 35. Ladite zéolithe est avantageusement composée de cristallites, de préférence isotropes, dont les tailles sont comprises entre 5 nm et 1 µm, de préférence entre 10 nm et 500 nm et de manière préférée entre 20 nm et 100 nm. La zéolithe initiale utilisée dans l'invention peut être sous forme brute de synthèse (avec un structurant organique) ou calcinée, de préférence, ladite zéolithe initiale est sous forme calcinée.

L'étape de désilication selon l'invention est mise en œuvre dans une solution alcaline, de préférence une solution aqueuse alcaline. Ladite solution alcaline est avantageusement une solution aqueuse alcaline à base d'hydroxyde de lithium LiOH, d'hydroxyde de sodium NaOH, d'hydroxyde de potassium KOH ou d'hydroxyde de césium CsOH. La solution alcaline a une normalité comprise entre 0,5 et 2 M, préférentiellement entre 0,7 et 1,5 M et de manière plus préférée entre 0,8 et 1,2 M.

Avantageusement, le rapport entre le volume (exprimé en mL) de la solution alcaline utilisée à l'étape i) de désilication et la masse de zéolithe sèche à désilicer (exprimé en g) est compris entre 2 et 20 et préférentiellement entre 4 et 18. Le mélange de la zéolithe et de la solution alcaline doit est placé sous agitation avec une vitesse d'agitation comprise entre 100 et 1000 tr/min, de préférence entre 200 et 400 tr/min, et chauffé à une température comprise entre 40 et 100°C, préférentiellement entre 50 et 100°C, de manière plus préférée entre 50 et 90°C et de manière encore plus préférée entre 60 et 90°C, pendant une durée comprise entre 5 min et 24 h, préférentiellement entre 15 min et 10 h, de manière plus préférée entre 15 min et 5 h et de manière encore plus préférée entre 30 min et 3 h.

### ii) Étape de lavage

A la suite de l'étape de désilication i) conduisant à la zéolithe modifiée, le mélange est avantageusement refroidi à température ambiante avant la mise en œuvre de l'étape ii) de lavage du procédé de préparation selon l'invention.

La zéolithe issue de l'étape i) est lavée avantageusement entre 2 et 10 fois avec de l'eau distillée. Le rapport entre le volume d'eau distillée (mL) utilisé et la masse de zéolithe (g) est typiquement compris entre 2 et 20, préférentiellement entre 4 et 18. La zéolithe lavée est avantageusement séparée par centrifugation, avec une vitesse comprise entre 2000 et 14000 tr/min, de préférence entre 5000 et 1000 tr/min. Des lavages successifs à l'eau distillée peuvent être effectués jusqu'à l'obtention d'un pH voisin de sept dans la solution aqueuse séparée par centrifugation. La zéolithe lavée est éventuellement séchée en étuve à une température comprise entre 60 et 130°C pour une durée comprise entre 1 h et 24 h. La zéolithe éventuellement séchée peut ensuite subir l'étape iv) du procédé de préparation, à savoir l'étape d'échange ionique. Dans une variante du procédé selon l'invention, ladite zéolithe est préalablement soumise à l'étape iii) de mise en forme avec une matrice de sorte que le pourcentage poids de la zéolithe soit compris entre 1 et 90% par rapport au poids du support, avant la mise en œuvre de l'étape iv) d'échange ionique.

### Traitement acide

Avantageusement selon l'invention, la zéolithe EUO lavée issue de l'étape ii) ou la zéolithe du support à l'issue de l'étape iii) subit un traitement acide par suspension dans une solution acide, de préférence une solution aqueuse acide, avant l'étape iv) d'échange ionique

Le traitement acide peut être réalisé avec toute solution aqueuse diluée d'acide minéral ou organique, comme l'acide chlorhydrique HCl, l'acide phosphorique H₃PO₄, l'acide nitrique HNO₃, l'acide citrique C₆H₈O₇, l'acide acétique C₂H₄O₂ ou encore l'acide sulfurique H₂SO₄ ayant une normalité comprise entre 0,001 et 2 M, préférentiellement entre 0,01 et 1,5 M, de manière plus préférée entre 0,02 et 1 M et de manière encore plus préférée entre 0,05 et 0,8 M. De préférence, le traitement acide est effectué avec une solution aqueuse diluée d'acide chlorhydrique. Le rapport entre le volume de la solution aqueuse utilisée pour réaliser le traitement acide (mL) et la masse de zéolithe lavée sèche ou la masse de support (zéolithe mise en forme) (g) sèche est compris entre 2 et 200 et préférentiellement entre 5 et 150. Le mélange est placé sous agitation avec une vitesse d'agitation comprise entre 100 et 1000 tr/min, de préférence entre 200 et 400 tr/min, et chauffé à une température comprise entre 30 et 100°C, préférentiellement entre 40 et 90°C, de manière plus préférée entre 45 et 85°C et de manière encore plus préférée entre 50 et 80°C, pendant une durée comprise entre 5 min et 24 h, préférentiellement entre 15 min et 10 h, de manière plus préférée entre 30 min et 8 h et de manière encore plus préférée entre 45 min et 8 h. A la fin du traitement acide, le mélange est avantageusement refroidi à température ambiante. La zéolithe ou le support issu de cette étape de lavage acide sont lavés avantageusement entre 2 et 10 fois avec de l'eau distillée. Le rapport entre le volume d'eau distillée (mL) utilisé et la masse de zéolithe ou de support (g) est typiquement compris entre 2 et 200, préférentiellement entre 5 et 150. La zéolithe lavée (lavage acide) est avantageusement séparée par centrifugation, avec une vitesse comprise entre 2000 et 14000 tr/min, de préférence entre 5000 et 1000 tr/min. Le support lavé (lavage acide) est avantageusement séparé par filtration. Des lavages successifs à l'eau distillée peuvent être effectués jusqu'à l'obtention d'un pH voisin de sept dans la solution aqueuse de lavage. La zéolithe lavée ou le support lavé (lavage acide) sont éventuellement séchés en étuve à une température comprise entre 60 et 130°C pour une durée comprise entre 1 h et 24 h.

### iii) Préparation du support

La préparation du catalyseur se poursuit par la mise en oeuvre de ladite étape iii) de préparation du support, encore appelée étape de mise en forme de la zéolithe lavée issue de l'étape ii) avec une matrice.

Pour la mise en oeuvre de ladite étape iii) de préparation du support par mise en forme de ladite zéolithe lavée de type structural EUO, de préférence de la zéolithe EU-1 lavée, issue de l'étape ii) on utilise une matrice pour la mise en forme choisie parmi les argiles, la magnésie, les alumines, les silices, l'oxyde de titane, l'oxyde de bore, la zircone, les phosphates d'aluminium, les phosphates de titane, les phosphates de zirconium, les silices-alumines et le charbon ou un mélange d'au moins deux de ces compositions. De préférence, la matrice est une alumine.

La teneur en zéolithe dans le support est comprise entre 3 et 80% poids et de manière plus préférée comprise entre 4 et 60% poids.

La préparation du support conformément à ladite étape iii) consiste plus particulièrement à malaxer la zéolithe lavée de type structural EUO, de préférence la zéolithe EU-1 lavée, issue de l'étape ii), dans un gel humide de matrice, préférentiellement d'alumine, obtenu généralement par mélange d'au moins un acide et d'une poudre de matrice, pendant une durée nécessaire à l'obtention d'une bonne homogénéité de la pâte, soit par exemple pendant une dizaine de minutes, puis à passer la pâte ainsi obtenue à travers une filière pour former des extrudés, par exemple de diamètre de 0,4 à 4 mm. Des billes peuvent également être obtenues par la technique de granulation. La mise en forme est généralement suivie d'un séchage puis d'une calcination. Le séchage est avantageusement réalisé à une température comprise entre 100 et 150°C pendant une durée comprise entre 5 et 20 h en étuve. La calcination est avantageusement réalisée à une température comprise entre 250 et 600°C pendant une durée comprise entre 1 et 8 h.

### iv) Etape d'échange ionique

Conformément à l'invention la zéolithe lavée issue de l'étape ii) ou le support issu de l'étape iii) sont soumis à un ou plusieurs échange(s) ionique(s) de manière à éliminer au moins en partie le cation alcalin présent en position cationique dans la zéolithe.

Une solution aqueuse contenant un précurseur d'ammonium tel que le nitrate d'ammonium NH₄NO₃, l'acétate d'ammonium CH₃COONH₄, l'hydroxyde de tétrapropylammonium C₁₂H₂₇NO, l'hydroxyde de tétraméthylammonium C₄H₁₃NO ou l'hydroxyde de tétraéthylammonium C₈H₂₁NO ayant une normalité comprise entre 0,2 et 12 M peut être utilisée pour réaliser l'étape iv) d'échange. Le rapport entre le volume de la solution aqueuse utilisée pour effectuer l'échange (mL) et la masse de zéolithe sèche ou de support sec à échanger (g) est compris entre 2 et 20 et préférentiellement entre 5 et 15. Le solide est versé dans un ballon ou un Erlenmeyer dans lequel se trouve une solution aqueuse avec le précurseur d'ammonium. Le mélange est placé sous agitation et éventuellement chauffé à une température comprise entre 50 et 100°C pendant une durée comprise entre 2 et 10 heures. La solution est ensuite enlevée avantageusement et le solide est rincé avec de l'eau distillée entre 2 et 10 fois puis éventuellement séché en étuve à une température comprise entre 60 et 130°C pour une durée comprise entre 1 et 24 h. L'étape d'échange peut être répétée plusieurs fois de manière à éliminer substantiellement tout cation alcalin éventuellement présent en position cationique dans la zéolithe. Le solide obtenu est ensuite avantageusement calciné pour une durée comprise entre 1 et 24 h, de préférence sous air sec, à une température comprise entre 300°C et 600°C, de préférence entre 450°C et 550°C dans un réacteur en quartz, équipé d'un fritté en son milieu, avec une circulation du gaz de bas en haut dont le débit est compris entre 0,5 L/h/g de solide et 3 L/h/g de solide.

La zéolithe de type structural EUO modifiée présente dans le catalyseur selon l'invention, se présente très avantageusement sous sa forme protonée (forme hydrogène H⁺). De préférence, la teneur en cation alcalin dans ladite zéolithe sous sa forme protonée est inférieure à 3000 ppm poids.

### v) Etape de dépôt du métal

L'étape v) de préparation du catalyseur consiste à déposer au moins un métal du groupe VIII de la classification périodique des éléments sur ladite zéolithe échangée issue de l'étape iv) ou ledit support échangé issu de l'étape iv) de sorte que le pourcentage poids dudit métal soit de 0,01 à 4%, de préférence de 0,05 à 2% par rapport au poids du catalyseur final.

Conformément à l'invention, ledit catalyseur comprend au moins un métal du groupe VIII choisi parmi le fer, le cobalt, le nickel, le ruthénium, le rhodium, le palladium, l'osmium, l'iridium et le platine, de préférence choisi parmi les métaux nobles du groupe VIII, de manière très préférée choisi parmi le palladium et le platine et de manière encore plus préférée le platine.

La dispersion du(es) métal(ux) du groupe VIII, déterminée par chimisorption, par exemple par titration H₂-O₂ ou par chimisorption du monoxyde de carbone, est comprise entre 50% et 100%, de préférence entre 60% et 100% et de manière encore plus préférée entre 70% et 100%. Le coefficient de répartition macroscopique du(es) métal(ux) du groupe VIII, obtenu à partir de son profil déterminé par microsonde de Castaing, défini comme le rapport des concentrations du(es) métal(ux) du groupe VIII au cœur du grain par rapport au bord de ce même grain, est compris entre 0,7 et 1,3, de préférence entre 0,8 et 1,2. La valeur de ce rapport, voisine de 1, témoigne de l'homogénéité de la répartition du(es) métal(ux) du groupe VIII dans le catalyseur.

Le catalyseur comprend éventuellement en outre au moins 0,01 à 2%, de préférence 0,05 à 1% poids par rapport au poids du catalyseur final d'un métal additionnel choisi dans le groupe formé par les métaux des groupes IIIA, IVA et VIIB de la classification périodique des éléments et de préférence choisi parmi le gallium, l'indium, l'étain et le rhénium. Ledit métal additionnel est de préférence choisi parmi l'indium, l'étain et le rhénium.

Pour le dépôt du métal du groupe VIII de la classification périodique des éléments, toutes les techniques de dépôt connues de l'homme du métier et tous les précurseurs de tels métaux peuvent convenir.

Lorsque le dépôt du (des) métal(ux) est effectué sur le support, le contrôle de certains paramètres mis en œuvre lors du dépôt, en particulier la nature du précurseur du (des) métal(ux) du groupe VIII utilisé(s), permet d'orienter le dépôt du(es)dit(s) métal(ux) majoritairement sur la matrice ou sur la zéolithe modifiée.

Ainsi, pour introduire le(s) métal(ux) du groupe VIII, préférentiellement le platine et/ou le palladium, majoritairement sur la matrice, on peut mettre en œuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur la matrice et le(s)dit(s) métal(ux) présente(nt) une bonne dispersion et une bonne répartition macroscopique à travers le grain de catalyseur.

On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur la zéolithe modifiée. Ainsi, dans le cas du platine, le précurseur peut être par exemple choisi parmi es composés ammoniaqués tels que les sels de platine (II) tétramines de formule Pt(NH₃)₄X₂, les sels de platine (IV) hexamines de formule Pt(NH₃)₆X₄ ; les sels de platine (IV) halogénopentamines de formule (PtX(NH₃)₅)X₃ ; les sels de platine N-tétrahalogénodiamines de formule PtX₄(NH₃)₂ ; et les composés halogénés de formule H(Pt(acac)₂X); X étant un halogène choisi dans le groupe formé par le chlore, le fluor, le brome et l'iode, X étant de préférence le chlore, et "acac" représentant le groupe acétylacétonate (de formule brute C₅H₇O₂), dérivé de l'acétylacétone. Avec de tels précurseurs, le(s) métal(ux) du groupe VIII est(sont) déposé(s) majoritairement sur la zéolithe et le(s)dit(s) métal(ux) présente(nt) une bonne dispersion et une bonne répartition macroscopique à travers le grain de catalyseur.

L'imprégnation à sec du métal du groupe VIII sur la zéolithe mise en forme (support) conduit au dépôt dudit métal à la fois sur la matrice et sur la zéolithe modifiée.

Dans le cas où le catalyseur préparé selon l'invention contient également au moins un métal choisi parmi les métaux des groupes IIIA, IVA et VIIB, toutes les techniques de dépôt d'un tel métal connues de l'homme du métier et tous les précurseurs de tels métaux peuvent convenir.

On peut ajouter le(s) métal(ux) du groupe VIII et celui(ceux) des groupes IIIA, IVA et VIIB, soit séparément soit simultanément dans au moins une étape unitaire. Lorsqu'au moins un métal des groupes IIIA, IVA et VIIB est ajouté séparément, il est préférable qu'il soit ajouté après le métal du groupe VIII.

Le métal additionnel choisi parmi les métaux des groupes IIIA, IVA et VIIB peut être introduit par l'intermédiaire de composés tels que par exemple les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Le métal additionnel choisi parmi les métaux des groupes IIIA, IVA et VIIB peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De plus, des traitements intermédiaires tels que par exemple une calcination et/ou une réduction peuvent être appliqués entre les dépôts successifs des différents métaux.

Une réduction préalable du catalyseur final ex *situ,* sous courant d'hydrogène, peut être mise en œuvre, par exemple à une température de 450°C à 600°C, pendant une durée de 0,5 à 4 h (heures).

Le catalyseur comprend éventuellement du soufre avec une teneur telle que le rapport du nombre d'atomes de soufre sur le nombre d'atome de(s) métal(ux) du groupe VIII soit compris entre 0,5 et 2.

Dans le cas où ie catalyseur ne contient pas de soufre, une réduction du métal sous hydrogène est réalisée *in situ* avant injection de la charge.

Dans le cas où le catalyseur préparé selon l'invention contient du soufre, le soufre est introduit sur le catalyseur mis en forme, calciné, contenant le ou les métaux cités précédemment, soit *in situ* avant la réaction catalytique, soit *ex situ.* La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

### Techniques de caractérisation

Le rapport Si/Al atomique global de la zéolithe EU-1 initiale est mesuré par fluorescence X. La fluorescence X est une technique d'analyse élémentaire globale qui permet l'analyse de tous les éléments du système périodique à partir du bore. Il est possible de doser de quelques ppm (partie par millions) jusqu'à 100%. Dans cette invention, cette technique est utilisée pour doser le silicium et l'aluminium de la zéolithe (en pourcentage massique) et permet ainsi de calculer le rapport atomique Si/Al. Les analyses FX de l'aluminium et du silicium ont été réalisées sur poudre sur un appareil Thermofischer Scientific Advant-X.

La teneur en sodium dans les différentes zéolithes sous leur forme protonée (après les étapes d'échange ioniques) est mesurée par spectrométrie d'absorption atomique ou spectrométrie d'émission optique. La teneur en sodium a été déterminée par un spectromètre d'émission optique (ICP-OES) SPECTRO, ARCOS SOP.

Les propriétés texturales de la zéolithe EUO initiale et des zéolithes EUO modifiées sont mesurées par adsorption/désorption d'azote à -196°C. La surface spécifique est calculée par la méthode BET. Le volume microporeux (V_{micro}) ainsi que la surface externe (Sₑₓₜ) sont obtenus par la méthode du "t-plot", l'épaisseur statistique étant calculée par la formule de Harkins et Jura. Le volume mésoporeux (V_{méso}) est obtenu en soustrayant le volume microporeux du volume poreux total. On entend par volume poreux total, le volume d'azote adsorbé pour une pression relative d'azote (P/P₀) de 0,95. Les mesures des isothermes d'adsorption-désorption des échantillons ont été effectuées sur le porosimètre Micromeritics ASAP 2024. L'opération de dégazage des solides est effectuée préalablement à l'aide d'une baie de prétraitement. Le dégazage se fait par une chauffe en deux temps : un premier palier de température de 2h à 100°C suivi d'un second palier de 6h à 500°C; les rampes de montée en température étant d'environ vingt degrés Celsius par minute.

La technique de diffraction des rayons X a été employée pour vérifier si la structure cristallographique de la zéolithe utilisée est bien présente dans les différents échantillons. Ceci est effectué par comparaison du diffractogramme expérimental avec le diffractogramme théorique (n° fiche JCPDS 04-007-2506). Les analyses ont été effectuées sur un diffractomètre PANanytical X'Pert PRO MPD θ-θ, en utilisant la raie Kα du cuivre (λ = 1,5402 Å) et un détecteur X'celerator. Lors de l'analyse le balayage a été fait entre 2° et 72° à température ambiante avec un pas de 0,02° et un temps de mesure de 6 secondes par pas.

La microscopie électronique à transmission (MET) à été utilisée pour évaluer la taille typique des cristallites de zéolithe. Les observations par MET ont été réalisées en utilisant un microscope JEOL 21000F - FEG (Field Emission Gun) avec une tension d'accélération de 200 kV.

### Mise en œuvre du catalyseur dans le procédé d'isomérisation de la coupe C8 aromatiques

La présente invention a également pour objet un procédé d'isomérisation d'une coupe aromatique contenant au moins un composé aromatique à huit atomes de carbone par molécule, ledit procédé comprenant la mise en contact de ladite coupe aromatique avec au moins ledit catalyseur préparé selon l'invention.

Ladite coupe aromatique contenant au moins un composé aromatique ayant huit atomes de carbone par molécule comprend en particulier comme composé aromatique ayant huit atomes de carbone par molécule soit uniquement un mélange de xylènes, soit uniquement de l'éthylbenzène, soit un mélange de xylène(s) et d'éthylbenzène.

Ledit procédé d'isomérisation est mis en oeuvre généralement selon les conditions opératoires suivantes :une température de 300°C à 500°C, de préférence de 320°C à 450°C et de manière encore plus préférée de 340°C à 430°C ; une pression partielle d'hydrogène de 0,3 à 1,5 MPa, de préférence de 0,4 et 1,2 MPa et de manière encore préférée de 0,7 à 1,2 MPa ; une pression totale de 0,45 à 1,9 MPa, de préférence de 0,6 à 1,5 MPa ; et une vitesse spatiale d'alimentation, exprimée en kilogramme de charge (coupe aromatique) introduite par kilogramme de catalyseur et par heure, de 0,25 à 30 h⁻¹, de préférence de 1 à 10 h⁻¹ et de manière encore préférée de 2 à 6 h⁻¹.

Les exemples suivants illustrent l'invention sans toutefois en limiter la portée.

### Exemple 1 (non conforme) : Préparation d'une zéolithe EU-1

On synthétise une zéolithe EU-1 conformément à l'enseignement du brevet EP-B1-0.042.226 en utilisant le structurant organique 1,6 N,N,N,N',N',N'-hexaméthylhexaméthylène diammonium. Pour la préparation d'une telle zéolithe, le mélange réactionnel présente la composition molaire suivante : 60 SiO₂ : 10,6 Na₂O : 5,27 NaBr : 1,5 Al₂O₃ : 19,5 Hexa-Br₂ : 2777 H₂O. Hexa-Br₂ étant le 1,6 N,N,N,N',N',N'-hexaméthylhexaméthylène diammonium, le brome étant le contre-ion. Le mélange réactionnel est placé dans un autoclave sous agitation (300 tours/min) pendant 5 jours à 180°C.

Cette zéolithe EU-1 subit tout d'abord une calcination dite sèche à 550°C sous flux d'air sec durant 10 heures de manière à éliminer le structurant organique. Le solide est ensuite mis sous reflux durant 4 heures dans une solution de nitrate d'ammonium (100 mL de solution par gramme de solide, concentration en nitrate d'ammonium de 10 M) afin d'échanger les cations alcalins par des ions ammonium. Cette étape d'échange est effectuée quatre fois. Le solide est ensuite calciné à 550°C pendant 4 h en four tubulaire. Le solide ainsi obtenu est référencé **EU-1(1)** et possède un rapport atomique Si/Al de 15 et une teneur en sodium de 73 ppm. L'analyse par Diffraction des Rayons X confirme que la zéolithe EU-1 a bien été obtenue. La microscopie électronique à transmission met en évidence la présence de cristallites d'une taille typiquement comprise entre 20 et 80 nm, ces cristallites formant des agglomérats. Ladite zéolithe EU-1(1) est analysée par porosimétrie à l'azote afin de déterminer ses caractéristiques texturales (tableau 1).

### Exemple 2 (invention) : Préparation d'une zéolithe EU-1 modifiée

La zéolithe EU-1(1) est placée dans un ballon à reflux équipé d'un agitateur mécanique où elle est soumise à un traitement alcalin dans les conditions suivantes :
- température de traitement : 85°C,
- concentration en soude de la solution aqueuse : 1 M,
- durée de traitement : 45 minutes,
- rapport volume de solution / masse de EU-1 (1) : 15 mL / g,
- vitesse de l'agitateur mécanique : 330 tr/min.

La zéolithe modifiée est ensuite lavée trois fois à l'eau distillée et séparée par centrifugation (8000 tr/min) puis séchée à l'étuve à 110°C pendant 12 h. A l'issue de ce traitement alcalin, on obtient une zéolithe modifiée notée **EU-1(2),** qui se trouve sous sa forme sodium. Le solide est mis sous reflux durant 4 heures dans une solution de nitrate d'ammonium (100 mL de solution par gramme de solide, concentration en nitrate d'ammonium de 10 M) afin d'échanger les cations alcalins par des ions ammonium. Cette étape d'échange est effectuée cinq fois. Le solide est ensuite calciné à 550°C pendant 4 h en four tubulaire. La zéolithe se trouve alors sous forme protonique et est nommée zéolithe **EU-1(3).** La teneur en sodium de la zéolithe est de 95 ppm et l'analyse par Diffraction des Rayons X confirme que les pics de diffraction caractéristiques de la zéolithe EU-1 sont toujours présents. Ladite zéolithe EU-1(3) est analysée par porosimétrie à l'azote afin de déterminer ses caractéristiques texturales (tableau 1).

### Exemple 3 (invention) : Préparation d'une zéolithe EU-1 modifiée

La zéolithe EU-1(2) obtenue à l'exemple 2 est placée dans un ballon où elle est soumise à un traitement acide dans les conditions suivantes :
- température de traitement : 65°C,
- concentration en acide chlorhydrique de la solution aqueuse : 0,1 M,
- durée de traitement : 360 minutes,
- rapport volume de solution / masse de EU-1(2) : 100 mL/g,
- vitesse de l'agitateur mécanique : 330 tr/min.

La zéolithe est ensuite lavée trois fois à l'eau distillée et séparée par centrifugation (8000 tr/min) puis séchée à l'étuve à 110°C pendant 12 h.

A l'issue de ce traitement acide, on obtient une zéolithe EU-1 modifiée **EU-1(4)** Le solide est mis sous reflux durant 4 heures dans une solution de nitrate d'ammonium (100 mL de solution par gramme de solide, concentration en nitrate d'ammonium de 10 M) afin d'échanger les cations alcalins par des ions ammonium. Cette étape d'échange est effectuée cinq fois. Le solide est ensuite calciné à 550°C pendant 4 h en four tubulaire. La zéolithe se trouve alors sous forme protonique et est nommée zéolithe **EU-1(5).** La teneur en sodium de ladite zéolithe est de 70 ppm et l'analyse par Diffraction des Rayons X confirme que les pics de diffraction caractéristiques de la zéolithe EU-1 sont toujours présents. Ladite zéolithe EU-1(5) est analysée par porosimétrie à l'azote afin de déterminer ses caractéristiques texturales (tableau 1).

### Exemple 4 (non conforme) : Préparation d'une zéolithe EU-1 modifiée

La zéolithe EU-1(1) est placée dans un ballon à reflux équipé d'un agitateur mécanique où elle est soumise à un traitement alcalin dans les conditions suivantes :
- température de traitement : 65°C,
- concentration en soude de la solution aqueuse : 0,05 M,
- durée de traitement : 60 minutes,
- rapport volume de solution / masse de EU-1 (1) : 15 mL / g,
- vitesse de l'agitateur mécanique : 330 tr/min.

La zéolithe modifiée est ensuite lavée trois fois à l'eau distillée et séparée par centrifugation (8000 tr/min) puis séchée à l'étuve à 110°C pendant 12 h.

A l'issue de ce traitement alcalin, on obtient une zéolithe modifiée notée **EU-1 (6),** qui se trouve sous sa forme sodium. Le solide est mis sous reflux durant 4 heures dans une solution de nitrate d'ammonium (100 mL de solution par gramme de solide, concentration en nitrate d'ammonium de 10 M) afin d'échanger les cations alcalins par des ions ammonium. Cette étape d'échange est effectuée cinq fois. Le solide est ensuite calciné à 550°C pendant 4 h en four tubulaire. La zéolithe se trouve alors sous forme protonique et est nommée zéolithe EU-1 (7), sa teneur en sodium étant de 92 ppm. L'analyse par Diffraction des Rayons X confirme que les pics de diffraction caractéristiques de la zéolithe EU-1 sont toujours présents. Ladite zéolithe **EU-1(7)** est analysée par porosimétrie à l'azote afin de déterminer ses caractéristiques texturales (tableau 1).

Le Tableau 1 présente les caractéristiques texturales des zéolithes EU-1(1) (parent) et EU-1 modifiées EU-1 (3), EU-1(5) et EU-1 (7) déterminées à partir des isothermes d'adsorption d'azote. La comparaison des propriétés texturales de la zéolithe EU-1 parent EU-1(1) avec la zéolithe EU-1(7) montre que la mise en œuvre d'un traitement alcalin de la zéolithe EU-1 dans des conditions non conformes ne permet pas de développer significativement la surface externe de la zéolithe parent ni d'augmenter le volume mésoporeux. A contrario, la mise en œuvre d'un traitement alcalin dans des conditions conformes à l'invention (EU-1(3)) permet d'augmenter la surface externe de 35 m²/g à 94 m²/g et d'augmenter le volume mésoporeux de 0,24 à 0,41 mL/g. La mise en oeuvre du traitement acide consécutif au traitement alcalin dans des conditions conformes à l'invention (EU-1(5)) permet encore d'augmenter la surface externe (pour atteindre une valeur de 160 m²/g) ainsi que le volume mésoporeux (pour atteindre une valeur de 0,59 mL/g). De plus le traitement acide permet de libérer de la microporosité, le volume microporeux atteignant une valeur de 0,103 mL/g contre 0,004 mL/g pour le solide ayant subi un traitement alcalin mais non traité à l'acide ensuite (EU-1(3)).

**Tableau 1 : Caractéristiques texturales des zéolithes EU-1(1) (parent) et EU-1 modifiées EU-1 (3), EU-1 (5) et EU-1 (7).**

| Référence de la zéolithe | S_{BET} (m²/g) | Sₑₓₜ (m²/g) | V_{méso} (mL/g) | V_{micro} (mL/g) |
|---|---|---|---|---|
| EU-1(1)(parent) | 456 | 35 | 0,24 | 0,150 |
| EU-1(3) | 103 | 94 | 0,41 | 0,004 |
| EU-1(5) | 398 | 160 | 0,59 | 0,103 |
| EU-1(7) | 175 | 39 | 0,21 | 0,061 |

### Exemple 5 (non conforme) : Préparation du catalyseur A comprenant une zéolithe EU-1 non modifiée

La zéolithe EU-1(1) (parent) de l'exemple 1 est malaxée avec un gel d'alumine de type SB3 fourni par la société Condéa-Sasol. La pate malaxée est extrudée au travers d'une filière de 1,4 mm. Après séchage en étuve une nuit à 110°C, les extrudés sont calcinés à 500°C durant deux heures (rampe de montée en température de 5°C/min) en lit traversé sous air sec (2 NL/h/g de solide). Les extrudés sont ensuite soumis à un échange anionique avec de l'acide hexachloroplatinique en présence d'acide chlorhydrique en tant qu'agent compétiteur, de manière à déposer 0,5% poids de platine par rapport au poids du catalyseur. Le solide humide est ensuite séché à 120°C pendant 12 heures et calciné sous un débit d'air sec à la température de 500°C pendant une heure.

Le catalyseur A ainsi obtenu contient, en poids, 10% de zéolithe EU-1 (1), 89,5% d'alumine et 0,5% de platine.

### Exemple 6 (invention) : Préparation du catalyseur B comprenant une zéolithe EU-1 modifiée

La zéolithe EU-1(3) obtenue à l'exemple 2 est malaxée avec un gel d'alumine de type SB3 fourni par la société Condéa-Sasol. La pate malaxée est extrudée au travers d'une filière de 1,4 mm. Après séchage en étuve une nuit à 110°C, les extrudés sont calcinés à 500°C durant deux heures (rampe de montée en température de 5°C/min) en lit traversé sous air sec (2 NL/h/g de solide). Les extrudés sont ensuite soumis à un échange anionique avec de l'acide hexachloroplatinique en présence d'acide chlorhydrique en tant qu'agent compétiteur, de manière à déposer 0,5% poids de platine par rapport au poids du catalyseur. Le solide humide est ensuite séché à 120°C pendant 12 heures et calciné sous un débit d'air sec à la température de 500°C pendant une heure.

Le catalyseur B ainsi obtenu contient, en poids, 10% de zéolithe modifiée EU-1(3), 89,5% d'alumine et 0,5% de platine.

### Exemple 7 (invention) : Préparation du catalyseur C comprenant une zéolithe EU-1 modifiée

La zéolithe EU-1 (5) obtenue à l'exemple 3 est malaxée avec un gel d'alumine de type SB3 fourni par la société Condéa-Sasol. La pate malaxée est extrudée au travers d'une filière de 1,4 mm. Après séchage en étuve une nuit à 110°C, les extrudés sont calcinés à 500°C durant deux heures (rampe de montée en température de 5°C/min) en lit traversé sous air sec (2 NL/h/g de solide). Les extrudés sont ensuite soumis à un échange anionique avec de l'acide hexachloroplatinique en présence d'acide chlorhydrique en tant qu'agent compétiteur, de manière à déposer 0,5% poids de platine par rapport au poids du catalyseur. Le solide humide est ensuite séché à 120°C pendant 12 heures et calciné sous un débit d'air sec à la température de 500°C pendant une heure.

Le catalyseur C ainsi obtenu contient, en poids, 10% de zéolithe modifiée EU-1(5), 89,5% d'alumine et 0,5% de platine.

### Exemple 8 (non conforme) : Préparation du catalyseur D comprenant une zéolithe EU-1 modifiée

La zéolithe EU-1(7) obtenue à l'exemple 4 est malaxée avec un gel d'alumine de type SB3 fourni par la société Condéa-Sasol. La pate malaxée est extrudée au travers d'une filière de 1,4 mm. Après séchage en étuve une nuit à 110°C, les extrudés sont calcinés à 500°C durant deux heures (rampe de montée en température de 5°C/min) en lit traversé sous air sec (2 NL/h/g de solide). Les extrudés sont ensuite soumis à un échange anionique avec de l'acide hexachloroplatinique en présence d'acide chlorhydrique en tant qu'agent compétiteur, de manière à déposer 0,5% poids de platine par rapport au poids du catalyseur. Le solide humide est ensuite séché à 120°C pendant 12 heures et calciné sous un débit d'air sec à la température de 500°C pendant une heure.

Le catalyseur D ainsi obtenu contient, en poids, 10% de zéolithe modifiée EU-1(7), 89,5% d'alumine et 0,5% de platine.

### Exemple 9 : Évaluation des propriétés catalytiques des catalyseurs A, B, C et D en isomérisation de l'éthylbenzène.

La charge, constituée uniquement d'éthylbenzène est isomérisée sur les différents catalyseurs en lit traversé dans un réacteur travaillant en isotherme et à hydrogène perdu. On évalue successivement les propriétés catalytiques des catalyseurs A, B, C et D pour l'isomérisation de l'éthylbenzène. Avant test catalytique, chaque catalyseur subit une étape de réduction sous débit d'hydrogène dans les conditions opératoires suivantes :
- pression totale : 1,3 MPa;
- débit d'hydrogène : 4 NL/h/g de catalyseur;
- montée de température ambiante à 450°C à 10°C/minute;
- palier d'une heure à 450°C;
- montée de 450°C à 480°C à 5°C/min;
- palier de deux heures à 480°C.

Après l'étape de réduction, la température et la pression sont ajustées aux valeurs de test visées, et la charge éthylbenzène est injectée. Les conditions opératoires de l'isomérisation sont les suivantes :
- température: 400°C;
- pression totale: 1 MPa;
- pression partielle d'hydrogène: 0,8 MPa;
- charge: éthylbenzène;
- vitesse spatiale d'alimentation, exprimée en kilogramme de charge introduite par kilogramme de catalyseur et par heure, égale à 10 h⁻¹.

Les catalyseurs ont été évalués en termes de conversion d'éthylbenzène et de sélectivité en xylènes.

La sélectivité en xylènes est calculée au moyen du rendement en xylènes produits. Le rendement en xylènes est déterminé à partir du pourcentage massique des xylènes produits, obtenu par analyse en ligne de chaque effluent par chromatographie en phase gazeuse (détecteur FID).

La conversion de l'éthylbenzène est le pourcentage d'éthylbenzène consommé.

**Tableau 2 : Conversion de l'éthylbenzène et sélectivité en xylènes sur les catalyseurs A. B. C et D après 4000 minutes de réaction**

| | catalyseur A | catalyseur B | catalyseur C | catalyseur D |
|---|---|---|---|---|
| conversion éthylbenzène(%) | 20,0 | 27,1 | 35,2 | 20,1 |
| sélectivité en xylènes (%) | 68,1 | 68,3 | 71,1 | 68,2 |
| rendement en xylènes (%) | 13,6 | 18,5 | 25,0 | 13,7 |

Les résultats présentés dans le tableau 1 montrent que les catalyseurs B et C comprenant chacun une zéolithe EU-1 modifiée selon le procédé décrit par l'invention, conduisent à de bien meilleures performances catalytiques en termes de conversion d'éthylbenzène que celles obtenues au moyen du catalyseur A comprenant une zéolithe EU-1 non modifiée et au moyen du catalyseur D comprenant une zéolithe EU-1 modifiée selon un procédé non conforme à l'invention. Les catalyseurs B et C selon l'invention sont donc plus actifs que le catalyseur A de l'état de la technique et que le catalyseur D. Par ailleurs les catalyseurs B et C selon l'invention présentent des sélectivités sensiblement comparables à celles des catalyseurs A et D. En conséquence, les catalyseurs B et C selon l'invention conduisent à un rendement en xylènes bien supérieur aux rendements en xylènes obtenus avec les catalyseurs comparatifs A et D.

## Revendications

1. Procédé de préparation d'un catalyseur d'isomérisation d'une coupe aromatique contenant au moins un composé aromatique ayant 8 atomes de carbone par molécule à partir d'une zéolithe initiale de type structural EUO ayant un rapport atomique Si/Al global compris entre 5 et 100, ledit catalyseur comprenant au moins une matrice et au moins un métal du groupe VIII, le procédé comprenant au moins les étapes suivantes :
i) une étape de désilication de ladite zéolithe initiale de type structural EUO par suspension dans une solution alcaline avec une normalité comprise entre 0,5 et 2 M, agitation avec une vitesse d'agitation comprise entre 100 et 1000 tr/min, et chauffage à une température comprise entre 40 et 100°C pendant une durée comprise entre 5 min et 24 h,
ii) une étape de lavage à l'eau distillée de la zéolithe issue de l'étape i),
iii) une étape de préparation du support par mise en forme de ladite zéolithe EUO lavée issue de l'étape ii) avec une matrice de sorte que le pourcentage poids de la zéolithe soit compris entre 3 et 80% par rapport au poids du support,
iv) au moins une étape d'échange ionique de la zéolithe lavée issue de l'étape ii) ou du support issu de l'étape iii), de manière à éliminer au moins en partie le cation alcalin présent en position cationique dans la zéolithe,
v) une étape de dépôt d'au moins un métal du groupe VIII de la classification périodique des éléments sur ladite zéolithe échangée issu de l'étape iv) ou ledit support échangé issu de l'étape iv) de sorte que le pourcentage poids dudit métal soit de 0,01 à 4% par rapport au poids du catalyseur,
procédé dans lequel la matrice pour la mise en forme du support est choisie parmi les argiles, la magnésie, les alumines, les silices, l'oxyde de titane, l'oxyde de bore, la zircone, les phosphates d'aluminium, les phosphates de titane, les phosphates de zirconium, les silices-alumines et le charbon ou un mélange d'au moins deux de ces compositions,
et dans lequel la solution alcaline est une solution aqueuse alcaline à base d'hydroxyde de lithium LiOH, d'hydroxyde de sodium NaOH, d'hydroxyde de potassium KOH ou d'hydroxyde de césium CsOH.

2. Procédé selon la revendication 1 dans lequel la zéolithe initiale de type structural EUO est choisie parmi la zéolithe EU-1, la zéolithe TPZ-3 et la zéolithe ZSM-50.

3. Procédé selon la revendication 1 ou 2 dans lequel ladite zéolithe initiale est sous forme calcinée.

4. Procédé selon l'une des revendications précédentes dans lequel, la zéolithe EUO lavée issue de l'étape ii) ou la zéolithe du support à l'issue de l'étape iii) subit un traitement acide par suspension dans une solution acide, de préférence une solution aqueuse acide, avant l'étape iv) d'échange ionique.

5. Procédé selon l'une des revendications précédentes dans lequel le métal du groupe VIII est choisi parmi le fer, le cobalt, le nickel, le ruthénium, le rhodium, le palladium, l'osmium, l'iridium et le platine.

6. Procédé selon l'une des revendications précédentes dans lequel catalyseur comprend en outre au moins 0,01 à 2% poids d'un métal additionnel choisi dans le groupe formé par les métaux des groupes IIIA, IVA et VIIB de la classification périodique des éléments.

7. Procédé d'isomérisation d'une coupe aromatique contenant au moins un composé aromatique à huit atomes de carbone par molécule, ledit procédé comprenant la mise en contact de ladite coupe aromatique avec au moins le catalyseur préparé selon l'une des revendications 1 à 6.

8. Procédé selon la revendication 7 mis en œuvre à une température de 300°C à 500°C; une pression partielle d'hydrogène de 0,3 à 1,5 MPa; une pression totale de 0,45 à 1,9 MPa; et une vitesse spatiale d'alimentation, exprimée en kilogramme de charge introduite par kilogramme de catalyseur et par heure de 0,25 à 30 h⁻¹.

9. Procédé selon la revendication 7 ou 8 dans lequel la coupe aromatique comprend comme composé aromatique ayant huit atomes de carbone par molécule soit uniquement un mélange de xylènes, soit uniquement de l'éthylbenzène, soit un mélange de xylène(s) et d'éthylbenzène.

## Patentansprüche

1. Verfahren zur Herstellung eines Katalysators zur Isomerisation eines aromatischen Schnitts, umfassend mindestens eine aromatische Verbindung mit 8 Kohlenstoffatomen pro Molekül aus einem Initialzeolithen vom Strukturtyp EUO mit einem Gesamtatomverhältnis Si/Al zwischen 5 und 100, wobei der Katalysator mindestens eine Matrix und mindestens ein Metall der Gruppe VIII umfasst, wobei das Verfahren mindestens die folgenden Schritte umfasst:
i) einen Schritt des Entsilizierens des Initialzeolithen vom Strukturtyp EUO durch Suspension in einer Alkalilösung mit einer Normalität zwischen 0,5 und 2 M, Umrühren mit einer Rührgeschwindigkeit zwischen 100 und 1000 U/min und Erhitzen auf eine Temperatur zwischen 40 und 100°C während einer Dauer zwischen 5 min und 24 h,
ii) einen Schritt des Waschens des aus dem Schritt i) stammenden Zeolithen mit destilliertem Wasser,
iii) einen Schritt der Herstellung des Trägers durch Formen des aus dem Schritt ii) stammenden gewaschenen Zeolithen EUO mit einer Matrix, so dass der Gewichtsprozentsatz des Zeolithen zwischen 3 und 80 % bezogen auf das Gewicht des Trägers beträgt,
iv) mindestens einen Schritt des Ionenaustauschs des aus dem Schritt ii) stammenden gewaschenen Zeolithen oder des aus dem Schritt iii) stammenden Trägers, um zumindest teilweise das an der Kationenposition im Zeolithen vorhandene Alkalikation zu beseitigen,
v) einen Schritt des Aufbringens mindestens eines Metalls der Gruppe VIII des Periodensystems der Elemente auf den aus dem Schritt iv) stammenden ausgetauschten Zeolithen oder den aus dem Schritt iv) stammenden ausgetauschten Träger, so dass der Gewichtsprozentsatz des Metalls 0,01 bis 4 % bezogen auf das Gewicht des Katalysators beträgt,
wobei bei dem Verfahren die Matrix zum Formen des Trägers unter Ton, Magnesiumoxid, Aluminiumoxiden, Siliziumoxiden, Titanoxid, Boroxid, Zikonoxid, Aluminiumphosphaten, Titanphosphaten, Zirkoniumphosphaten, Silizium-Aluminiumoxiden und Kohle oder einem Gemisch mindestens zweier dieser Zusammensetzungen ausgewählt ist, und bei dem die Alkalilösung eine wässrige Alkalilösung auf Basis von Lithiumhydroxid LiOH, Natriumhydroxid NaOH, Kaliumhydroxid KOH oder Cäsiumhydroxid CsOH ist.

2. Verfahren nach Anspruch 1, bei dem der Initialzeolith vom Strukturtyp EUO unter dem Zeolithen EU-1, dem Zeolithen TPZ-3 und unter dem Zeolithen ZSM-50 ausgewählt ist.

3. Verfahren nach Anspruch 1 oder 2, bei dem der Initialzeolith in kalzinierter Form vorhanden ist.

4. Verfahren nach einem der vorhergehenden Ansprüche, bei dem der aus dem Schritt ii) stammende gewaschene Zeolith oder der Zeolith des Trägers aus dem Schritt iii) einer Säurebehandlung durch Suspension in einer Säurelösung, vorzugsweise einer wässrigen Säurelösung, vor dem Schritt iv) des Ionenaustauschs unterzogen wird.

5. Verfahren nach einem der vorhergehenden Ansprüche, bei dem das Metall der Gruppe VIII unter Eisen, Kobalt, Nickel, Ruthenium, Rhodium, Palladium, Osmium, Iridium und Platin ausgewählt ist.

6. Verfahren nach einem der vorhergehenden Ansprüche, bei dem der Katalysator ferner mindestens 0,01 bis 2 Gew.-% eins zusätzlichen Metalls umfasst, das in der Gruppe ausgewählt ist, die von den Metallen der Gruppen IIIA, IVA und VIIB des Periodensystems der Elemente gebildet ist.

7. Verfahren zur Isomerisation einer aromatischen Fraktion, umfassend mindestens eine aromatische Verbindung mit acht Kohlenstoffatomen pro Molekül, wobei das Verfahren die Kontaktnahme der aromatischen Fraktion mit mindestens dem nach einem der Ansprüche 1 bis 6 hergestellten Katalysator umfasst.

8. Verfahren nach Anspruch 7, das bei einer Temperatur von 300 °C bis 500 °C, einem teilweisen Wasserstoffdruck von 0,3 bis 1,5 MPa, einem Gesamtdruck von 0,45 bis 1,9 MPa, einer Zuspeisungs-Raumgeschwindigkeit, ausgedrückt in Kilogramm eingeführter Fraktion pro Kilogramm Katalysator und pro Stunde, von 0,25 bei 30 h⁻¹ eingesetzt wird.

9. Verfahren nach Anspruch 7 oder 8, bei dem der aromatische Schnitt als aromatische Verbindung mit acht Kohlenstoffatomen pro Molekül entweder nur ein Xylol-Gemisch oder nur ein Ethylbenzol-Gemisch oder nur ein Gemisch von Xylol(en) und Ethylbenzol umfasst.

## Claims

1. A process for the preparation of a catalyst for the isomerization of an aromatic cut containing at least one aromatic compound containing eight carbon atoms per molecule from an initial zeolite with structure type EUO having an overall Si/Al atomic ratio in the range 5 to 100, said catalyst comprising at least one matrix and at least one metal from group VIII, said process comprising at least the following steps:
i) a step for desilication of said initial zeolite with structure type EUO by suspension in an alkaline solution with a normality in the range 0.5 to 2M, stirring at a stirring speed in the range 100 to 1000 rpm, and heating to a temperature in the range 40°C to 100°C for a period in the range 5 min to 24 h;
ii) a step for washing the zeolite obtained from step i) with distilled water;
iii) a step for preparation of a support by shaping said washed EUO zeolite obtained from step ii) with a matrix in a manner such that the percentage by weight of the zeolite is in the range 3% to 80% with respect to the weight of the support;
iv) at least one step for ion exchange of the washed zeolite obtained from step ii) or the support obtained from step iii) in a manner such as to eliminate at least a portion of the alkaline cation present in the cationic position in the zeolite;
v) a step for depositing at least one metal from group VIII of the periodic classification of the elements onto said exchanged zeolite obtained from step iv) or said exchanged support obtained from step iv) in a manner such that the percentage by weight of said metal is 0.01% to 4% with respect to the weight of the catalyst,
process in which the matrix for shaping the support is selected from clays, magnesia, aluminas, silicas, titanium oxide, boron oxide, zirconia, aluminium phosphates, titanium phosphates, zirconium phosphates, silica-aluminas and carbon, or a mixture of at least two of these compositions,
and in which said alkaline solution is an aqueous alkaline solution based on lithium hydroxide LiOH, sodium hydroxide NaOH, potassium hydroxide KOH or caesium hydroxide CsOH.

2. The process according to claim 1, in which the initial zeolite with structure type EUO is selected from EU-1 zeolite, TPZ-3 zeolite and ZSM-50 zeolite.

3. The process according to claim 1 or claim 2, in which said initial zeolite is in the calcined form.

4. The process according to one of the preceding claims, in which the washed EUO zeolite obtained from step ii) or the zeolite of the support obtained from step iii) undergoes an acid treatment by suspension in an acidic solution, preferably an aqueous acidic solution, before the ion exchange step iv).

5. The process according to one of the preceding claims, in which the metal from group VIII is selected from iron, cobalt, nickel, ruthenium, rhodium, palladium, osmium, iridium and platinum.

6. The process according to one of the preceding claims, in which the catalyst further comprises at least 0.01% to 2% by weight of an additional metal selected from the group formed by metals from groups IIIA, IVA and VIIB of the periodic classification of the elements.

7. A process for the isomerization of an aromatic cut containing at least one aromatic compound containing eight carbon atoms per molecule, said process comprising bringing said aromatic cut into contact with at least the catalyst prepared in accordance with one of claims 1 to 6.

8. The process according to claim 7, carried out at a temperature of 300°C to 500°C, a partial pressure of hydrogen of 0.3 to 1.5 MPa; a total pressure of 0.45 to 1.9 MPa; and a feed space velocity, expressed in kilograms of feed introduced per kilogram of catalyst per hour, of 0.25 to 30 h⁻¹.

9. The process according to claim 7 or claim 8, in which the aromatic cut comprises, as the aromatic compound containing eight carbon atoms per molecule, either uniquely a mixture of xylenes, or uniquely ethylbenzene, or a mixture of xylene(s) and ethylbenzene.
